# EUROPÄISCHE PATENTSCHRIFT

(11) **EP 3 846 883 B1**
(45) Veröffentlichungstag und Bekanntmachung des Hinweises auf die Patenterteilung: **12.10.2022**
(21) Anmeldenummer: 19787158.5
(22) Anmeldetag: 29.08.2019
(51) Int. Cl.: A61M 13/00, A61M 11/00

(54) **MEDIZINISCHES INSTRUMENT ZUM GERICHTETEN EINBRINGEN EINER SUBSTANZ IN EINEN HOHLRAUM EINES KÖRPERS UND WERKZEUG DAFÜR**
MEDICAL INSTRUMENT FOR THE TARGETED INTRODUCTION OF A SUBSTANCE INTO A BODY CAVITY, AND TOOL THEREFOR
INSTRUMENT MÉDICAL POUR L'ADMINISTRATION CIBLÉE D'UNE SUBSTANCE DANS UNE CAVITÉ CORPORELLE ET OUTIL À CET EFFET

(30) Priorität: 04.09.2018 DE 102018121513
(43) Veröffentlichungstag der Anmeldung: 14.07.2021
(73) Patentinhaber: Prof. Reymond & Hetzel GbR, 78667 Villingendorf (DE)
(72) Erfinder: HETZEL, Alexander, 78667 Villingendorf (DE); REYMOND, Marc, 72076 Tübingen (DE)
(74) Vertreter: Mehl-Mikus, Claudia
(86) Internationale Anmeldenummer: PCT/EP2019/000248
(87) Internationale Veröffentlichungsnummer: WO 2020/048626

(56) Entgegenhaltungen:
- WO-A1-2013/063404
- DE-C2- 3 320 076
- DE-U- 7 309 776
- JP-A- 2001 104 490
- US-A1- 2002 151 873
- US-B1- 6 319 248

## Beschreibung

Die Erfindung betrifft ein medizinisches Instrument zum gerichteten Einbringen einer Substanz in einen Hohlraum eines Körpers und ein Werkzeug dafür.

Aus dem Stand der Technik sind allgemein Verbindungsmöglichkeiten wie ein Luer-Lock-Anschluss für feste, lösbare Verbindungen von Werkzeugen und Fluidleitungen für medizinischen Anwendungen bekannt, so auch aus DE 20 2009 003 255 U1.

Ferner sind medizinische Instrumente bekannt, in denen ein Fluid geführt wird. DE 73 09 776 U offenbart einen Katheter, der an seinem distalen Ende einen Kopf aufweist, an dem gleichmäßig verteilte Öffnungen vorliegen, aus denen ein Fluid austreten kann, um den Katheter per Rückstoß gezielt zu positionieren.

In DE 33 20 076 A1 ist ein Düsenkörper mit einer Mehrzahl radial angeordneter Düsen für ein medizinisches Instrument gezeigt, der in einem Schaft vorliegt, drehbar gelagert und mit einem Fluid führenden Schlauch fest verbunden ist. Die Drehbewegung ergibt sich durch den Rückstoß des durch die Düsen austretenden Fluids und ist auf eine Welle übertragbar.

Auch sind Werkzeuge für medizinische Instrumente bekannt, die dafür ausgebildet sind, Substanzen, insbesondere therapeutische Substanzen in einen Hohlraum eines Körpers zu bringen.

Die WO 20121163 346 A1 beschreibt ein Trokarsystem mit einem Werkzeug, das an seinem distalen Ende eine Düse aufweist, wobei das distale Ende in eine Körperhöhle hineinragen kann. Mittels dieser Düse kann bspw. in einem Pneumoperitoneum eine therapeutische Substanz versprüht werden. Die Sprührichtung dieser Düse ist allerdings nur in eine Richtung gerichtet, so dass die Substanz nicht gleichmäßig innerhalb des Bauchraums versprüht werden kann.

Aus US 2002/0151873 A1 ist ein weiteres laparoskopisches Werkzeug bekannt, das am distalen Ende eines röhrenförmigen Schafts einen Sprühkopf mit einer Vielzahl zu einer Seite gerichteten Sprühöffnungen angeordnet ist. Über ein Kugelgelenk kann die Ausrichtung des Sprühkopfs eingestellt werden.

WO 2013/063404 A1 offenbart eine Sprühvorrichtung zum Ausbringen eines Gewebeklebers auf einer Gewebeoberfläche, insbesondere der Nasennebenhöhlen, wobei ein Sprühkopf am distalen Ende einer Kanüle angeordnet ist und mehrere in unterschiedlichen Winkeln und Ebenen angeordnete Sprühöffnungen aufweist, sodass der Kleber in einem halbkugelförmigen Sprühmuster in der Körperhöhle ausgebracht werden kann.

Ausgehend von diesem Stand der Technik ist es Aufgabe der vorliegenden Erfindung, ein verbessertes Werkzeug für ein medizinisches Instrument zum gerichteten Einbringen einer Substanz in einen Hohlraum eines Körpers bereitzustellen, das ein gleichmäßiges Versprühen der jeweiligen Substanz in dem Körperhohlraum ermöglicht.

Diese Aufgabe wird durch ein Werkzeug mit den Merkmalen des Anspruchs 1 gelöst.

Die weitere Aufgabe, ein medizinisches Instrument bereitzustellen, das einfach aufgebaut und kostengünstig ist, wird durch das Instrument mit den Merkmalen des unabhängigen Anspruchs 7 gelöst.

Weiterbildungen des Werkzeugs und des medizinischen Instruments sind in den Unteransprüchen ausgeführt.

Eine erste Ausführungsform des Werkzeugs bezieht sich auf ein Werkzeug für ein medizinisches Instrument zum gerichteten Einbringen einer Substanz in einen Hohlraum eines Körpers. Es kann darunter jede Art von Körperhohlraum verstanden werden, bevorzugter Einsatz kann in einem Pneumoperitoneum sein. Als Substanz kommt jede fluide therapeutische Substanz, wie ein Medikament, Medikament in Lösung oder auch eine simple Spüllösung in Frage; sie muss selbst oder in einer geeigneten Lösung versprüht werden können.

Das Werkzeug weist einen Schaft mit einem Lumen auf, wobei an dem distalen Ende des Schafts ein Düsenkopf mit zumindest zwei Düsen angeordnet ist. Dabei sind die Düsen in Bezug zu einer Mittelachse des Schafts in einem Winkel von weniger als 180° und gleichmäßig voneinander beabstandet. Vorteilhaft sind die Düsen gleichmäßig an dem Düsenkopf verteilt und bilden in Bezug zu der Mittelachse des Schafts bzw. des Werkzeugs eine symmetrische Anordnung.

Dabei weisen die Düsen des Werkzeugs Düsenöffnungen auf, die über einen Verteilerkonus mit einer Bohrung und dem Lumen des Schafts verbunden sind. Das Lumen befindet sich innerhalb des Schafts und wird durch dessen innere Bohrung gebildet, der als Fluidzugang zu den Düsen dient. Durch den Verteilerkonus werden die Düsen gleichmäßig mit der fluiden Substanz beaufschlagt. Die Bohrung kann als Mittelbohrung oder auch außermittig ausgebildet sein.

Erfindungsgemäß ist ferner der Düsenkopf in dem Schaft derart drehbar gelagert, dass der Düsenkopf bei Beaufschlagung der Düsen mit der Substanz durch die gleichmäßige Beaufschlagung der Düsen aus dem Verteilerkonus und die erfindungsgemäße Anordnung der Düsen auf dem Düsenkopf ein Drehmoment erfährt. Dazu kann eine Stirnfläche des distalen Endes des Schafts korrespondierend zu einer Form des Düsenkopfes ausgebildet sein. Um drehbar zu sein, ist der Düsenkopf rotationssymmetrisch zu der Mittelachse des Schafts bzw. des Werkzeugs. In einer bevorzugten Variante kann der Düsenkopf rund, im Wesentlichen kugelförmig sein. Er kann aber alternativ auch zylindrisch sein. Andere Formen sind ebenfalls möglich. Die Stirnfläche des Schafts kann (in einem Längsschnitt betrachtet) dabei flach, abgerundet, teilkugelförmig, konusförmig oder in einer anderen Form ausgebildet sein.

Ferner kann das Werkzeug vorsehen, dass der Düsenkopf drei Düsen aufweist. Im Falle von drei Düsen schließen ihre Fluidachsen zueinander und in Bezug auf die Werkzeug-Mittelachse jeweils einen Winkel α in einem Bereich von 90° bis 180°, bevorzugt von 100° bis 140°, besonders bevorzugt von 120° ein. Dieser Winkel wird ferner auch zwischen den einzelnen Düsen eingeschlossen, so dass im bevorzugten Fall zwischen den Mittelachsen aller drei Düsen und einem gedachten Mittelpunkt des Düsenkopfes (ausgehend von der Mittelachse des Werkzeugs) der gleiche Winkel α = 120° vorliegt. In einer weiteren Ausführungsform des Werkzeugs können zwei bis zehn Düsen kreisförmig, ggf. auf verschiedenen Achsen angeordnet sein. Bevorzugt ist der Winkel α = 360 Grad / Anzahl der Düsen. Diese Symmetrie erzeugt den größtmöglichen Abstand zwischen den Düsen und sorgt für eine räumlich gesehen gute und gleichmäßige Verteilung der versprühten Substanz.

Der Düsenkopf kann durch ein generatives Fertigungsverfahren hergestellt werden und passgenau auf den Schaft abgestimmt sein. In einer bevorzugten Ausführungsform beträgt ein Außendurchmesser des Schafts ca. 10 mm. Diese Abmessung passt in bestehende Trokarsysteme hinein, wobei auch Durchmesser in einem Bereich von 1 mm bis 30 mm möglich sind.

In einer Weiterbildung des Werkzeugs kann der Düsenkopf einen zylindrischen Körper aufweisen, der in dem Lumen des Schafts aufgenommen ist. Der Düsenkörper kann eine Ringnut aufweisen, in der bspw. ein Gleitdichtring aufgenommen werden kann. Der Düsenkörper kann so in dem Lumen drehbar gelagert werden, ohne zu lecken. In einer weiteren Ausführungsform weist der Düsenkörper in einem proximalen Abschnitt ein Schaufelrad mit einer Vielzahl Flügel auf und in einem distalen Abschnitt, der an den Düsenkopf angrenzt, einen Lagerring, der dazu dient, den Düsenkörper in dem Schaft des Werkzeugs zu halten. Dieser Düsenkörper ist so in dem Schaft eingesetzt, dass das Schaufelrad (bzw. dessen Position) in einer Montageanordnung des Werkzeugs mit einer Durchgangsöffnung mit zwei Lumen in dem Schaft korrespondiert, durch die Fluid fließen kann. Durch Auftreffen des Fluids auf die Flügel des Schaufelrads wird der Düsenkörper in Rotation versetzt. Das gesamte Fluid kann durch die Lumen, über das Schaufelrad und an die Mittelbohrung und schließlich die Düse des Werkzeugs geleitet werden. Zwischen dem Schaufelrad und dem Lagerring ist eine zylindrische Gleitfläche vorgesehen, an der die Komponenten reibungsarm gleiten können. Der Schaft weist eine zu dem Düsenkörper korrespondierende Ausnehmung auf, um insbesondere den Lagerring aufnehmen zu können. Der Kopf kann somit einfach in Drehung versetzt werden, und gleichzeitig sicher in dem Werkzeug gehalten werden.

Um das Werkzeug mit einem Trokar eines Trokarsystems, einem Handstück bzw. einer Handhabe oder anderen bestehenden Instrumentarien verbinden zu können und eine Fluidquelle anschließen zu können, kann der Schaft an seinem proximalen Ende einen Anschluss zur fluidischen Verbindung des Lumens mit einer Fluidquelle aufweisen, wobei der Anschluss ein Luer-Lock-Anschluss ist. Es sind aber auch andere Verbindungen möglich.

Die Erfindung bezieht sich ferner auf ein medizinisches Instrument zum Einbringen von Fluiden in einen Hohlraum eines Körpers. Das Instrument kann ein Trokar, eine Fluidquelle sowie ein Werkzeug aufweisen, wobei das Werkzeug mit dem Trokar lösbar und mit der Fluidquelle fluidisch verbunden werden kann. Verwendet wird das erfindungsgemäße Werkzeug.

Erfindungsgemäß ist das Werkzeug, wenn es mit dem Trokar innerhalb eines Trokarsystems verwendet wird, mit der Fluidquelle über eine flexible Fluidleitung verbunden, wobei die Fluidquelle mit dem Werkzeug lösbar verbunden werden und das Werkzeug in den Trokar des medizinischen Instruments eingesteckt werden kann. Ebenso kann eine Handhabe vorgesehen sein, in die das Werkzeug eingesteckt werden kann. Durch Betätigen von entsprechenden Betätigungselementen an der Handhabe bzw. dem Trokar kann das Werkzeug mit der zu versprühenden Substanz beaufschlagt werden, indem eine fluidische Verbindung zwischen der Fluidquelle und dem Werkzeug hergestellt wird. Die fluide Substanz gelangt durch das Lumen zu dem Düsenkörper, wird durch die Mittelbohrung des Düsenkörpers zu dem Verteilerkonus geleitet und dort über die Zuführleitungen zu den Düsenöffnungen der Düsen. Die Substanz tritt aus den Düsenöffnungen aus und wird versprüht. Durch die gleichmäßige Beaufschlagung der Düsen aus dem Verteilerkonus und der erfindungsgemäßen Anordnung der Düsen auf dem Düsenkopf, erfährt der Düsenkopf bei einem Flüssigkeitsdruck in einem Bereich von 1 bar bis 100 bar ein Drehmoment und fängt an zu rotieren, wenn er mit der fluiden Substanz beaufschlagt wird. Folge ist eine gleichmäßige Verteilung der Substanz in dem jeweiligen Körperhohlraum; eine rein punktuelle Versprühung wird vermieden. Es muss nicht das gesamte Werkzeug rotiert werden, sondern nur der Kopf rotiert von alleine.

Weitere Ausführungsformen des Werkzeugs und des medizinischen Instruments sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Die Figuren sind lediglich eine schematische Darstellung einer Ausführungsform der Erfindung.

Dabei zeigen:
- **Fig. 1**: eine schematische Seitenansicht des erfindungsgemäßen Werkzeugs,
- **Fig. 2**: eine Längsschnittansicht A-A durch das Werkzeug,
- **Fig. 3**: eine Detailansicht B des Düsenkopfes im Längsschnitt,
- **Fig. 4**: eine Schnittansicht einer weiteren Ausführungsform des Düsenkopfes,
- **Fig. 5**: eine schematische Draufsicht auf das distale Ende des Düsenkopfes,
- **Fig. 6**: eine schematische Ansicht einer Handhabe mit eingestecktem Werkzeug, und
- **Fig. 7**: eine schematische Ansicht eines Trokars mit eingestecktem Werkzeug.

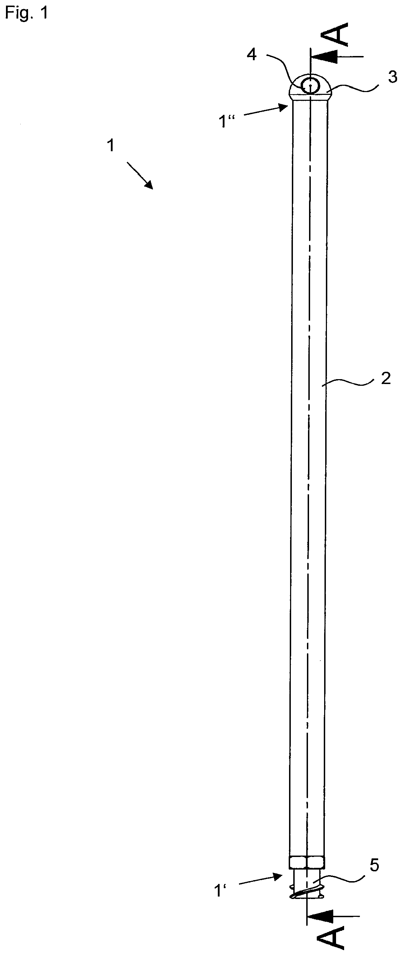

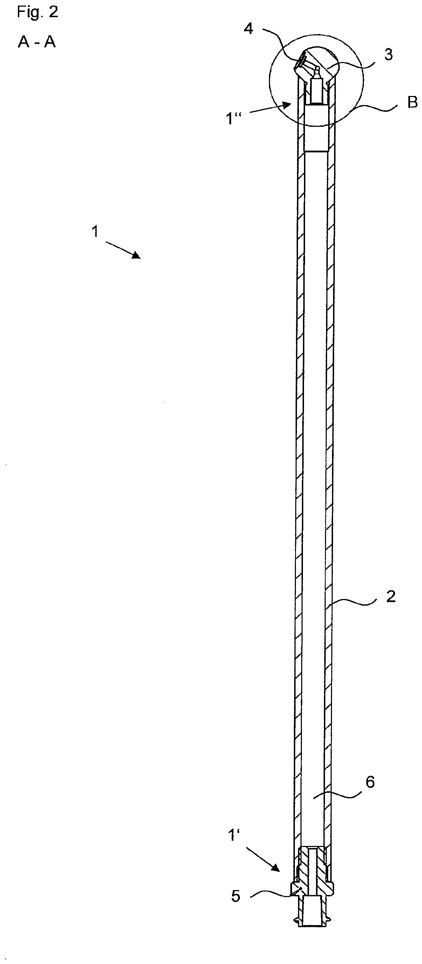

Die erfindungsgemäße Vorrichtung bezieht sich auf ein medizinisches Instrument, das ein Werkzeug 1 aufweist, das nach **Fig. 1** einen Schaft 2 mit einem proximalen Ende 1' und einem distalen Ende 1" hat. Die **Fig. 1** bis 3 zeigen das Werkzeug 1 in einzelnen Details. Am proximalen Ende 1' ist ein Anschluss, ein sog. Luer-Lock 5 angebracht, an den verschiedene Schläuche oder Flüssigkeitszugänge angeschlossen werden können. Der Luer-Lock 5 steht mit dem Lumen 6 fluidisch in Verbindung (siehe Schnittansicht A-A in Fig. **2**). Der Schaft 2 ist länglich und rohrfömig ausgebildet und hat in einer bevorzugten Ausführungsform einen Durchmesser von in etwa 10 Millimetern. Es sind Durchmesser in einem Bereich von 1 mm bis 30 mm möglich.

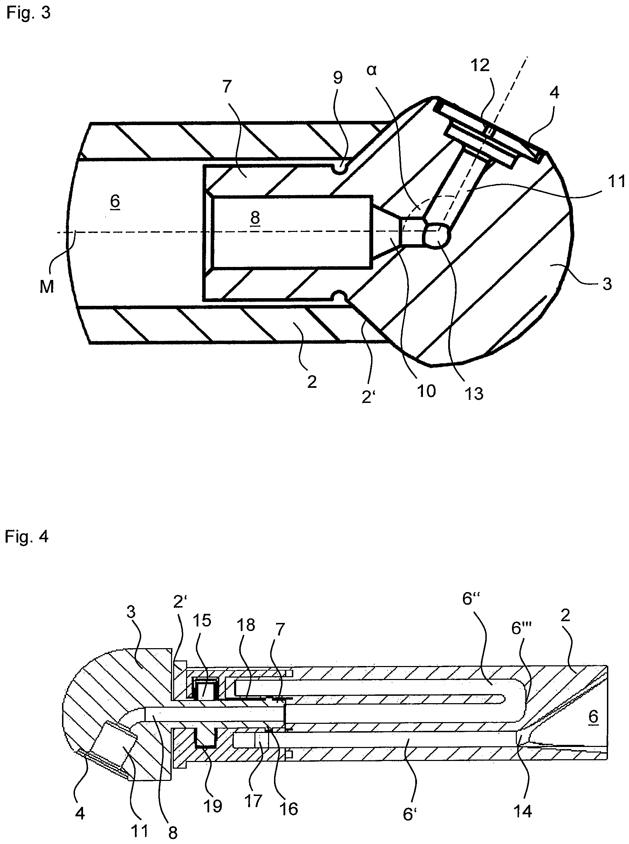

Der Schaft 2 weist ferner an seinem distalen Ende 1" einen Düsenkopf 3 mit mehreren Düsen 4 auf. So ist in **Fig. 3** eine Detailansicht B auf den Düsenkopf 3 dargestellt. Der Düsenkopf 3 weist einen Düsenkörper 7 mit einer Kavität sowie einer Ringnut 9 auf, die kurz unterhalb einer kugelförmigen Verdickung des Düsenkopfes 3 angeordnet ist. Die Kavität ist als Bohrung 8 bzw. Mittelbohrung ausgebildet und ist fluidisch mit einem Verteilerkonus 10 verbunden. Ein Zuführkanal 11 führt in einem Winkel α von in etwa 120° in Bezug zu einer Mittelachse M der Bohrung 8 von der Bohrung 8 weg und verbindet diese fluidisch mit einer Düsenöffnung 12. Zwischen Verteilerkonus 10 und Zuführkanal 11 ist eine kugelförmige Sammelkavität 13 vorgesehen, die auch den Mittelpunkt des Düsenkopfes 3 bildet. Die Bohrung 8 ist mit dem Lumen 6 des Schafts 2 fluidisch verbunden. Die Mittelachse M der Bohrung 8 als Mittelbohrung entspricht dabei der Mittelachse M des Schafts 2 und folglich auch des Werkzeugs 1.

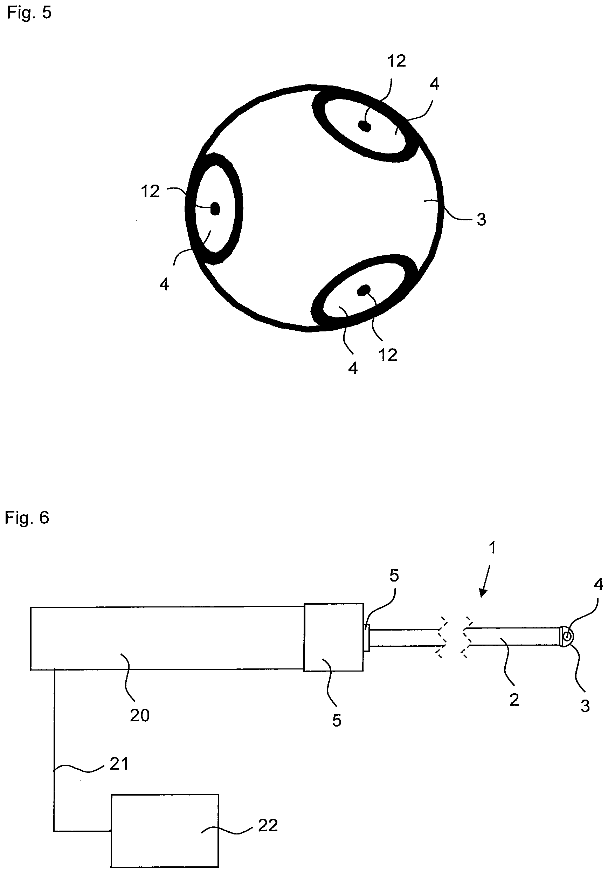

Der Kopf 3 ist im Wesentlichen kugelförmig gestaltet und weist, wie **Fig. 5** zeigt, insgesamt drei Düsen 4 auf. Die Düsen 4 sind über den Kugelkörper 3 gleichmäßig verteilt und schließen jeweils einen Winkel von 120° zum Mittelpunkt des Kopfes 3 ein. Der Kopf des Werkzeugs kann beliebig zwei bis zehn Düsen aufweisen, die kreisförmig zueinander, ggf. auf verschiedenen Achsen, angeordnet sein können.

Dadurch und besonders durch die in Fig. 5 gezeigte Form wird eine besonders gleichmäßige Verteilung des durch die Düse 4 versprühten Fluids erreicht. Der Mittelpunkt des Düsenkopfes 3 entspricht dabei einer räumlichen Mitte der Sammelkavität 13.

Fluid, das von dem Lumen 6 über die Bohrung 8 in den Verteilerkonus 10 gelangt, wird dort auf den folgenden reduzierten Querschnitt geleitet und von der Sammelkavität 13 an die einzelnen Zuführkanäle 11 geleitet und dann der jeweiligen Düsenöffnung 12 zugeführt.

Der Kopf 3 ist drehbar, wie aus **Fig. 3** und **4** ersichtlich. Eine Stirnfläche 2' des distalen Endes 1" des Schafts 2 ist hierzu korrespondierend ausgebildet, so dass der Düsenkopf 3 in dem distalen Ende 1" des Schafts 2 gelagert ist. Die Stirnseite 2' des Schafts 2 ist in **Fig. 3** konisch und nimmt den Kopf 3 teilweise auf.

In **Fig. 4** ist der Kopf 3 an seiner Seite, die dem distalen Ende 1" und damit der Stirnfläche 2' zugewandt ist, flach ausgebildet, korrespondierend zu der Stirnfläche 2', die hier ebenfalls flach ist. Damit der Düsenkörper 7 während der Benutzung nicht aus dem Schaft 2 herausgedrückt wird, weisen der Düsenkörper 7 und der Schaft 2 folgende Komponenten auf: Das Lumen 6 des Schafts 2 verjüngt sich zum distalen Ende 1" des Werkzeugs 1 hin und mündet in einen Konus 14, der das Lumen 6 mit einem Lumen 6' verbindet, dessen Durchmesser im Vergleich zu dem Lumen 6 stark verjüngt ist. Der Konus 14 ist in **Fig. 4** in einem unteren Drittel des Schafts 2 angeordnet, wobei das Lumen 6' parallel zur Mittelachse geführt ist. Das Lumen 6' weist in seinem distalen Endbereich eine Durchgangsöffnung 17 auf, durch die Fluid aus dem Lumen 6' auf ein Schaufelrad 16 geleitet werden kann. Der Düsenkörper 7 weist eine Vielzahl an Flügeln auf, die das Schaufelrad 16 bilden. Über das Schaufelrad 16 wird das Fluid in ein weiteres Lumen 6" geleitet, und über einen Bogen 6‴ in die Bohrung 8 des Düsenkörpers 7 Von dort führt die Bohrung 8 in den Zuführkanal 11 und zur Düse 4. Am Düsenkörper 7 ist ferner ein Lagerring 15 angeordnet, der in dem Schaftkörper 2 drehbar gelagert ist. Dazu ist im Schaftkörper 2 eine korrespondierende kreiszylindrische Ausnehmung 19 als Aufnahme ausgebildet. Damit wird der Düsenkörper 7 im Schaft 2 gehalten. Zwischen Lagerring 15 und Schaufelrad 16 ist eine Gleitfläche 18 vorgesehen, damit der Düsenkörper 7 in dem Schaft 2 reibungsarm sich drehen kann.

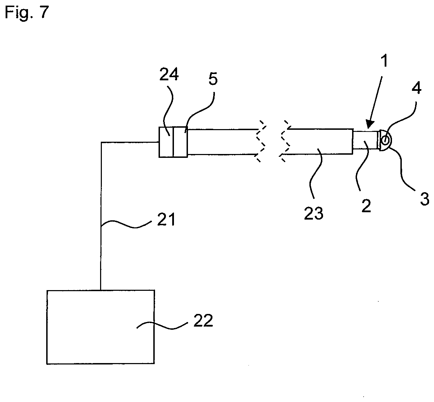

Das Werkzeug 1 kann über den Luer-Lock-Anschluss 5 mit einer Handhabe 20 und einer Fluidquelle 22 verbunden werden, wie **Fig. 6** zeigt. Die Fluidquelle 22 ist über eine Fiuidleitung 21 fluidisch mit der Handhabe 20 und damit dem Werkzeug 1 verbunden. Ferner kann das Werkzeug 1 auch zusammen mit einem Trokarsystem im Rahmen der minimalinvasiven Chirurgie verwendet werden, wie **Fig. 7** zeigt. Dazu wird das Werkzeug 1 in einen Trokar 23 gesteckt. Mittels eines anschließbaren Fluidanschlusses 24 kann das Werkzeug 1 über eine Fluidleitung 21 fluidisch mit der Fluidquelle 22 verbunden werden.

### BEZUGSZEICHENLISTE

- 1: Werkzeug
- 1': Proximales Ende
- 1": Distales Ende
- 2: Schaft
- 2': Stirnfläche
- 3: Düsenkopf
- 4: Düse
- 5: Luer-Lock Anschluss
- 6, 6', 6": Lumen
- 6‴: Bogen
- 7: Düsenkörper
- 8: Bohrung
- 9: Ringnut
- 10: Verteilerkonus
- 11: Zuführkanal
- 12: Düsenöffnung
- 13: Sammelkavität
- 14: Konus
- 15: Lagerring
- 16: Schaufelrad
- 17: Durchgangsöffnung
- 18: Gleitfläche
- 19: Ausnehmung

- 20: Handhabe
- 21: Fluidleitung
- 22: Fluidquelle
- 23: Trokar
- 24: Fluidanschluss

- A-A: Längsschnitt
- B: Kopf im Detail
- M: Mittelachse

## Patentansprüche

1. Werkzeug (1) für ein medizinisches Instrument zum gerichteten Einbringen einer Substanz in einen Hohlraum eines Körpers,
wobei das Werkzeug (1) einen Schaft (2) mit einem Lumen (6) aufweist, an dessen distalem Ende (1") ein Düsenkopf (3) mit zumindest zwei Düsen (4) angeordnet ist, wobei die Düsen (4) in Bezug zu einer Mittelachse (M) des Schafts (2) in einem Winkel von weniger als 180° (α) und gleichmäßig voneinander beabstandet sind, und Düsenöffnungen (12) aufweisen,
**dadurch gekennzeichnet, dass**
die Düsenöffnungen (12) über einen Verteilerkonus (10) mit einer Bohrung (8) und dem Lumen (6) verbunden sind,
wobei der Düsenkopf (3) in dem Schaft (2) drehbar gelagert ist und bei Beaufschlagung der Düsen (4) mit der Substanz ein Drehmoment erfährt.

2. Werkzeug (1) nach Anspruch 1,
**dadurch gekennzeichnet, dass**
der Düsenkopf (3) drei Düsen (4) aufweist, die in Bezug zu einer Mittelachse (M) des Schafts (2) in einem Winkel von 120° (α) und gleichmäßig voneinander beabstandet sind.

3. Werkzeug (1) nach Anspruch 1 oder 2,
**dadurch gekennzeichnet, dass**
eine Stirnfläche (2') des distalen Endes (1") des Schafts (2) korrespondierend zu einer Form des Düsenkopfes (3) ausgebildet ist.

4. Werkzeug (1) nach zumindest einem der Ansprüche 1 bis 3,
**dadurch gekennzeichnet, dass**
der Düsenkopf (3) einen zylindrischen Düsenkörper (7) aufweist, der in dem Lumen (6) des Schafts (2) aufgenommen ist,
wobei der Düsenkörper (7) eine Ringnut (9) aufweist, in der ein Gleitdichtring aufnehmbar ist.

5. Werkzeug (1) nach Anspruch 4,
**dadurch gekennzeichnet, dass**
der Düsenkörper (7) in einem proximalen Abschnitt ein Schaufelrad (16) mit einer Vielzahl Flügel aufweist und in einem distalen Abschnitt, der an den Düsenkopf (3) angrenzt, einen Lagerring (15) aufweist, und
wobei der Schaft (2) zwei Lumen (6', 6") aufweist, die über einen Bogen (6‴) miteinander verbunden sind und je eine Durchgangsöffnung (17) aufweisen, die in einer Montageanordnung des Werkzeugs (1) mit der Position des Schaufelrads (16) übereinstimmt.

6. Werkzeug (1) nach zumindest einem der Ansprüche 1 bis 5,
**dadurch gekennzeichnet, dass**
der Schaft (2) an seinem proximalen Ende (1') einen Anschluss (5) zur fluidischen Verbindung des Lumens (6) mit einer Fluidquelle (22) aufweist, wobei der Anschluss (5) ein Luer-Lock-Anschluss ist.

7. Medizinisches Instrument zum Einbringen von Fluiden in eine Körperhöhle, mit einem Trokar (23), einer Fluidquelle (22) sowie einem Werkzeug, das mit einer Handhabe (20) lösbar und mit der Fluidquelle (22) fluidisch verbindbar ist,
**dadurch gekennzeichnet, dass**
das Werkzeug ein Werkzeug (1) nach zumindest einem der Ansprüche 1 bis 6 ist.

8. Medizinisches Instrument nach Anspruch 7,
**dadurch gekennzeichnet, dass**
das Werkzeug (1) mit der Fluidquelle (22) über eine flexible Fluidleitung (21) verbunden ist.

## Claims

1. Tool (1) for a medical instrument for targeted introduction of a substance into a body cavity,
wherein the tool (1) comprises a shaft (2) with a lumen (6) having at its distal end (1") a nozzle head (3) with at least two nozzles (4), wherein the nozzles (4) are at an angle of less than 180° (α) in relation to a center axis (M) of the shaft (2) and are uniformly spaced apart from each other and comprise nozzle openings (12),
**characterized in that**
the nozzle openings (12) are connected by a distributor cone (10) to a bore (8) and to the lumen (6),
wherein the nozzle head (3) is rotatably supported in the shaft (2) and experiences a torque when the nozzles (4) are supplied with the substance.

2. Tool (1) according to claim 1,
**characterized in that**
the nozzle head (3) comprises three nozzles (4) which are at an angle of 120° (α) in relation to a center axis (M) of the shaft (2) and are uniformly spaced apart from each other.

3. Tool (1) according to claim 1 or 2,
**characterized in that**
an end face (2') of the distal end (1") of the shaft (2) is embodied so as to correspond to a shape of the nozzle head (3).

4. Tool (1) according to at least one of the claims 1 to 3,
**characterized in that**
the nozzle head (3) comprises a cylindrical nozzle body (7) which is received in the lumen (6) of the shaft (2),
wherein the nozzle body (7) comprises an annular groove (9) in which an annular sliding seal can be received.

5. Tool (1) according to claim 4,
**characterized in that**
the nozzle body (7) comprises in a proximal section an impeller (16) with a plurality of vanes and a bearing ring (15) in a distal section which adjoins the nozzle head (3), and
wherein the shaft (2) comprises two lumina (6', 6") that are connected by a bend (6‴) to each other and each comprise a through opening (17) which coincides with the position of the impeller (16) in a mounting arrangement of the tool (1).

6. Tool (1) according to at least one of the claims 1 to 5,
**characterized in that**
the shaft (2) at its proximal end (1') comprises a connector (5) for connecting in fluid communication the lumen (6) to a fluid source (22), wherein the connector (5) is a Luer-lock connector.

7. Medical instrument for introduction of fluids into a body cavity, with a trocar (23), a fluid source (22) as well as a tool that is connectable detachably to a handle (20) and in fluid communication to the fluid source (22),
**characterized in that**
the tool is a tool (1) according to at least one of the claims 1 to 6.

8. Medical instrument according to claim 7,
**characterized in that**
the tool (1) is connected to the fluid source (22) by a flexible fluid conduit (21).

## Revendications

1. Outil (1) pour un instrument médical destiné à l'introduction ciblée d'une substance dans une cavité corporelle,
l'outil (1) présentant une tige (2) avec un lumen (6), à l'extrémité distale (1") de laquelle est disposée une tête de buses (3) avec au moins deux buses (4), les buses (4) étant espacées de manière régulière et d'un angle inférieur à 180° (α) par rapport à un axe médian (M) de la tige (2), et présentant des orifices de buses (12), **caractérisé en ce que**
les orifices de buses (12) sont reliés à un trou (8) et au lumen (6) par un cône de répartition (10),
la tête de buses (3) étant montée sur pivot dans la tige (2) et faisant l'objet d'un couple de rotation lors de l'alimentation des buses (4) avec la substance.

2. Outil (1) selon la revendication 1,
**caractérisé en ce que**
la tête de buses (3) présente trois buses (4) qui sont espacées de manière régulière et d'un angle de 120° (α) par rapport à un axe médian (M) de la tige (2).

3. Outil (1) selon la revendication 1 ou 2,
**caractérisé en ce que**
une face frontale (2') de l'extrémité distale (1") de la tige (2) est conçue de manière à correspondre à une forme de la tête de buses (3).

4. Outil (1) selon au moins à l'une des revendications 1 à 3,
**caractérisé en ce que**
la tête de buses (3) présente un corps de buse cylindrique (7) qui est logé dans le lumen (6) de la tige (2),
le corps de buse (7) présentant une rainure annulaire (9) dans laquelle peut être logé un joint d'étanchéité coulissant.

5. Outil (1) selon la revendication 4,
**caractérisé en ce que**
le corps de buse (7) présente, dans une section proximale, une roue à aubes (16) avec un grand nombre de rotors et, dans une section distale qui est adjacente à la tête de buses (3), une bague de palier (15) et
la tige (2) présentant deux lumens (6', 6") qui sont reliés entre eux par un arc (6‴) et présentant respectivement une ouverture de passage (17) qui correspond, dans une disposition du montage de l'outil (1), à la position de la roue à aubes (16).

6. Outil (1) selon au moins à l'une des revendications 1 à 5,
**caractérisé en ce que**
la tige (2) présente, à son extrémité proximale (1'), un raccord (5) pour la liaison fluidique du lumen (6) avec une source de fluide (22), le raccord (5) étant un raccord Luer-Lock.

7. Instrument médical destiné à l'introduction de fluides dans une cavité corporelle, avec un trocart (23), une source de fluide (22) et un outil qui peut être détaché au moyen d'une poignée (20) et relié fluidiquement à la source de fluide (22),
**caractérisé en ce que**
l'outil est un outil (1) selon au moins à l'une des revendications 1 à 6.

8. Instrument médical selon la revendication 7,
**caractérisé en ce que**
l'outil (1) est relié à la source de fluide (22) par un conduit de fluide flexible (21).
